Europäisches Patentamt

(19) European Patent Office

Office européen des brevets

(11) Publication number: **0 302 697**
**A1**

# EUROPEAN PATENT APPLICATION

(12)

(21) Application number: **88307119.3**

(22) Date of filing: **02.08.88**

(51) Int. Cl.⁴: **C 07 C 57/075**
**C 07 C 51/50**

(30) Priority: **07.08.87 US 83543**

(43) Date of publication of application:
**08.02.89 Bulletin 89/06**

(84) Designated Contracting States: **DE ES FR GB IT**

(71) Applicant: **ROHM AND HAAS COMPANY**
**Independence Mall West**
**Philadelphia Pennsylvania 19105 (US)**

(72) Inventor: **Day, James Clarence**
**English Village Apartments Bldg. 5, Apt. A-3**
**North Wales Pennsylvania 19454 (US)**

**Bauer, William, Jr.**
**2046 Winthrop Road**
**Huntington Valley Pennsylvania 19006 (US)**

(74) Representative: **Tanner, James Percival et al**
**ROHM AND HAAS (UK) LTD. European Operations Patent**
**Department Lennig House 2 Mason's Avenue**
**Croydon CR9 3NB (GB)**

(54) Inhibition of polymerization of unsaturated acid in the vapor phase.

㉗ The formation of unwanted polymer in the vapour phase during the distillation of unsaturated acid is inhibited by adding to the distillation unit, during the distillation, 0.001 to 0.1 weight percent, based on the weight of the acid, of non-metallic nitrite salt or nitrite ester.

EP 0 302 697 A1

Description

## "INHIBITION OF POLYMERIZATION OF UNSATURATED ACID IN THE VAPOR PHASE"

This invention is concerned with an improved process for the distillation of unsaturated acids, and more specifically to improvements in inhibiting the formation of insoluble "popcorn" polymer of an unsaturated aliphatic acid in the distillation column, without introducing undesirable inhibitors or contaminants to the product, the residue, or the distillation equipment.

Polymerization of unsaturated acids in the vapor phase may occur under distillation conditions to produce, at a rapid rate, an insoluble "popcorn" polymer which may rapidly block the orifices of the column. Many of the inhibitors used to prevent polymerization in the liquid phase in the pot or boiler, the column, and the takeoff and collection lines are effective only in the liquid phase, not being volatile enough to inhibit polymerization in the vapor phase.

Unsaturated acids are those monomers with a terminal $CH2=C-$ group, which form polymers or oligomers under exposure to free-radical sources; on long standing; or on exposure to heat or a combination of these factors. Monomers with internal unsaturation, such as crotonic acid or linoleic acid, are excluded from this category. The acid may contain other functional groups, i.e. sites of reactivity. Examples of unsaturated acids falling within the scope of this invention include acrylic acid, methacrylic acid, ethacrylic acid, alpha-phenylacrylic acid, itaconic acid, ($C_1$ to $C_6$) monoalkyl itaconates, acryloxypropionic acid, alpha-hydroxymethylacrylic acid, and alpha-chloroacrylic acid.

GB-A-1,265,419 discloses gaseous nitric oxide (NO) as an inhibitor; the NO being supplied at a 10 to 15% concentration in nitrogen. The concurrent presence of phenothiazine in the reflux in the distillation column is also disclosed. There are inherent difficulties with this system, including the difficulty of adding a gas mixture at a controlled rate to the pot and column, and the need to prevent oxidation of NO to $NO_2$, because $NO_2$ promotes polymerization of acrylic acid and discolors the acrylic acid.

Japanese Kokai 74-100029 discloses using sodium nitrite in combination with p-aminophenol to prevent polymerization of methacrylic acid in a sealed tube. There is no disclosure that other unsaturated acids would not be polymerized. The metal salts create a disposal problem. Such salts are not soluble at high concentrations and foul equipment. In the presence of sulfuric acid or other strong acids often present in the crude acid, the metal salts may form insoluble sulfates which can foul equipment. Wastes from pot residues cannot be disposed of readily; for example, burning of the wastes leaves inorganic ash which is an additional cost of disposal.

Japanese Kokai 75-01009 and US-A-3,964,979 disclose metal nitrites, such as $NaNO_2$, or NO as inhibitors for vinyl aromatic monomers, including addition to distillation columns. Apart from the problems discussed above, it is not predictable from current inhibition theory that an effective inhibitor for one monomer will be effective for another. For example, Leplyanin et al, Izv. Akad. Nauk SSSR, Ser. Khim. 4, 754 (1975) demonstrates weak inhibition of methyl methacrylate polymerization with NO, but strong inhibition of styrene and vinyl acetate polymerization.

U.S.-A-2,819,296 discloses that "popcorn" polymerization in a tube reactor, in which methacrylic acid or acrylic acid are reacted with ethylene or propylene oxides to form the corresponding hydroxyalkyl esters, is not easily inhibited by "the usual stabilizing agents", but is prevented by alkali metal nitrites. U.S.-A-3,987,090 discloses that such "popcorn" polymerization can be avoided by conducting the same reaction in the presence of alkyl or alkenyl nitrites. This latter U.S. patent does not suggest that the alkyl or alkenyl nitrites would be effective in vapor phase inhibition or in the distillation of either reactant or product.

Dicyclohexylammonium nitrite is commercially sold and advertised as a corrosion inhibitor, useful in preventing oxidative attack on iron and other metals; there is no suggestion or teaching that this material could inhibit polymerization .

U.S.-A-2,874,099 discloses NO and salts of nitrous acid with an organic nitrogen base as stabilizers of unsaturated aldehydes. Examples of the salts are monosubstituted amines, such as cyclohexylammonium nitrite, quaternary ammonium salts, such as benzyltrimethylammonium nitrite, and (preferred) salts of secondary ammonium nitrites, such as dicyclohexylammonium nitrite. Also disclosed is the inhibition of formation of insoluble polymer during the distillation of acrolein and methacrolein. Further disclosed is the combination of such salts with hydroquinone in processing. There is no teaching or suggestion of these materials being useful as inhibitors in monomers of differing reactivity toward self-polymerization, such as acrylic or methacrylic acid.

Japanese Kokai 77-125116 discloses that adding metal nitrite salts to methyl methacrylate with hydroquinone lowers the polymerization residue on distillation.

The present invention may, in certain embodiments, enable the provision of one or more of the following: (i) an improved method for inhibiting the formation of "popcorn" polymer during the distillation of unsaturated acids by eliminating the need for a controlled feed of an inhibiting gas; (ii) the elimination of metallic residues which cause major disposal problems; and (iii) the provision of a combination of inhibitors which further improve the yield of acid.

According to the present invention, there is provided a process for the distillation of unsaturated acids, usually under reduced pressure, with inhibition of unwanted polymer formation in the vapor phase by charging from about 0.001 to about 0.1 weight percent of non-metallic nitrite ester inhibitor, e.g. alkyl nitrite, or non-metallic nitrite salt, e.g.

ammonium nitrite or substituted ammonium nitrite, to the distillation unit, preferably the distillation pot or the lower plates of the distillation column or both, during the distillation. Adding phenothiazine or hydroquinone in the distillation pot, preferably in an amount at least equimolar with that of the nitrite salt or ester, e.g. the alkyl nitrite or nitrite salt, will further increase the yield of the desired distilled unsaturated acid.

The inhibitors used in this invention are useful in preparing unsaturated monomers such as acrylic acid, methacrylic acid, ethacrylic acid, itaconic acid, acryloxypropionic acid, alpha-phenyl acrylic acid, mono($C_1$ to $C_6$)alkyl itaconates, alpha hydroxymethacrylic acid, alpha chloroacrylic acid and other alpha, beta-unsaturated monomers which may undergo unwanted polymerization to form insoluble polymer from the vapor phase during distillation. Acrylic acid is the most reactive toward unwanted "popcorn" polymerization.

The distillation may be carried out in all-glass equipment or in the presence of metal or ceramics inert to the polymerizable unsaturated acids and any sulfuric acids present. Pressure in the range of 50 to 200 mm. may be used. It is most convenient to distil at pressures in the range of from about 50 to about 100 mm., which gives a distillation range for effective fractionation well above room temperature, yet the unsaturated acid does not become overheated. The temperature is generally in the range of 70° to 100°C with a range of 80° to 90°C being preferred.

The unsaturated acid may contain water, especially if it is to be used later in aqueous systems. The monomer after distillation may be reinhibited against polymerization prior to packaging and storage. The resulting acid may be directly converted tp derivatives or polymerized. Such derivatives include alkyl esters and functionalized esters, for example, the hydroxyalkyl derivatives. Polymers may include polymers of high acid content or copolymers where the acid is present at a low level, such as 0.5 to 2 wt %. The resulting polymers have a variety of uses, for example, as coatings or thickeners.

The distillation may be batch, semi-continuous or continuous. The inhibitor system taught herein is easily renewed and controlled, and is especially effective in long distillation runs, such as continuous or semi-continuous processes.

The concentration of nitrite employed relative to unsaturated acid is preferably from about 0.001 to 0.003 percent by weight, based on the weight of the unsaturated acid.

The nitrite salts include substituted and unsubstituted ammonium nitrites wherein the nitrogen of the ammonium group alone may have from I to 4 ($C_1$ to $C_{10}$) alkyl, ($C_3$ to $C_6$) cycloalkyl, or aryl ($C_1$ to $C_6$) alkyl substituents. Illustrative examples are ammonium nitrite, monomethylammonium nitrite, trimethylammonium nitrite, tetraethylammonium nitrite, benzyltrimethylammonium nitrite, and dicyclohexylammonium nitrite. Some of the nitrite salt may be converted to ammonium acrylate, which does not cause the precipitation or disposal problems found for the metallic nitrite residues. Both the ammonium nitrite and the alkyl nitrite residues are fully combusted to volatile products in waste disposal equipment.

The nitrite esters include alkyl nitrites, for example, ($C_6$ to $C_{10}$) alkyl nitrites. Alkyl nitrites, which may be represented by the formula RONO wherein R represents the alkyl group, can be regarded as esters of nitrous acid. The volatility of the alkyl nitrite is a controlling factor in the selection of appropriate compounds. Isoamyl nitrite is too volatile to be effective in the lower portion of the distillation column. Too long an alkyl group diminishes volatility and availability of nitrite groups. Alkyl nitrites with $C_6$ to $C_{10}$ alkyl groups are preferred; $C_8$ is especially preferred. Illustrative examples include n-hexyl nitrite, n-heptyl nitrite, 1-octyl nitrite, 2-octyl nitrite, 1-nonyl nitrite, and 4-decyl nitrite.

The continuous addition of both nitrite inhibitors and a liquid phase antioxidant is preferred, especially in a semi-continuous or continuous distillation where crude acrylic acid or other relatively polymerizable unsaturated acids is added during part or all of the distillation cycle.

One or more free radical inhibitors in the liquid phase may be present in addition to the nitrite inhibitor.

There are a wide variety of inhibitors against polymerization in the liquid phase which may be useful in combination with the nitrites of the present invention. Some are effective in the absence of oxygen, such as quinones, while others, for example phenothiazine and hydroquinone, are more effective when oxygen is present. The latter type are preferred in commercial practice. Other useful inhibitors include alkyl, halo- and aryl-substituted phenothiazines where there is no substitution on nitrogen, naphthoquinone, and hydroquinones substituted at one or more carbons with alkyl or halo groups. Mixtures of such inhibitors may also be used. Their presence with the nitrites used in this invention assures inhibition in both the liquid and vapor phases. The use of the other inhibitors also appears to prevent wastage of the nitrites in side reactions, especially in forming $NO_2$, a known initiator of polymerization and a contributor to color formation.

The present invention will now be further illustrated by way of the following examples which are for illustrative purposes only and are not be construed as imposing any limitation on the scope of the invention.

Example I - Inhibition by Ammonium Nitrite

Acrylic acid (500g) is charged to a batch distillation unit (5-necked, 1-liter flask surmounted by a 10-plate, 2.54 cn (1 inch) Oldershaw column and distillation head). The acid (plus some water) is distilled at 200 mm pressure, resulting in flask and head temperatures of 105°C and 90°C, respectively. During the distillation, acrylic acid is added from a dropping funnel to maintain a constant liquid level in the flask and constant temperatures throughout the unit.

Ammonium nitrite is most conveniently handled as an aqueous solution stabilized against decomposition to nitrogen and water by the inclusion of ammonium hydroxide to bring the pH of the solution to 8.0.

A solution of 0.11 wt. % ammonium nitrite in $H_2O$ is fed to the flask at a rate of 10 ml/hr. (0.172 mmole nitrite/hr.). A solution of 2.0 wt. % each of phenothiazine (PTZ) and hydroquinone (HQ) in acrylic acid is fed at the top of the distilling head condenser at a rate of 10 ml/hr. (1.04 and 1.35 mmole/hr. of PTZ and HQ, respectively). The flask contents are sparged with air at a rate equivalent to 13.2 mmoles $O_2$/hr. Approximately 120 ml/hr. of acrylic acid-$H_2O$ is collected overhead. Under these conditions the system remained polymer-free during a run time of 4 hrs. and 20 mins.

### Example 2 - Preparation and Use of Tetrabutylammonium Nitrite

Silver nitrite (1g, 0.0065 moles) was dissolved in 175 ml of water. To this solution was added tetrabutylammonium bromide (2.1g, 0.0065 moles) in 25 ml of water dropwise with stirring over a 15 minute period at ambient temperatures. A silver bromide precipitate formed immediately. The mixture was stirred for 5 minutes after completion of the addition and then gravity filtered. To 50g of the filtrate was added 25g of water to produce a 0.63 wt. % aqueous tetrabutylammonium nitrite solution which was used as an inhibitor feed to a batch distillation of acrylic acid as described below.

Acrylic acid (AA) (500g) was charged to a batch distillation unit of Example 1. The acid (plus some water) was distilled at 300mmHg pressure, producing flask and head temperatures of 115° and 100°C, respectively.

The above prepared solution of 0.63 wt. % tetrabutylammonium nitrite was fed to the flask at a rate of 10 ml/hr. (0.219 mmole nitrite/hr.). A solution of 1.0 wt. % each of PTZ and HQ in AA was fed at the top of the distilling head condenser at a rate of 20 ml/hr. (1.04 and 1.35 mmole/hr. of PTZ and HQ, respectively). The flask content was sparged with air (13.2 mmole $O_2$/hr.). Approximately 120 ml/hr. of AA-$H_2O$ was collected overhead. After 5 hrs. of distillation only a few isolated specks of polymer were observed in the distillation head.

### Example 3 - Preparation and Use of Benzyltriethylammonium Nitrite

Silver nitrite (1g, 0.0065 moles) was dissolved in 175 ml of water. To this solution was added benzyltriethylammonium chloride (1.48g, 0.0065 moles) in 25 ml of water dropwise with stirring at ambient temperature over a 15 minute period. A silver chloride precipitate formed immediately. The mixture was stirred for 5 minutes after completion of the addition and then gravity filtered. To 50g of the filtrate was added 24g of water to produce a 0.52 wt. % aqueous benzyltriethylammonium nitrite solution. This was used as an inhibitor feed to a batch distillation of AA as described below.

Acrylic acid (500g) was distilled as in Example 2. PTZ, HQ and air were present as in Example 2. The above prepared solution of 0.52 wt. % benzyltriethylammonium nitrite was fed to the flask at a rate of 10 ml/hr. (0.219 mmole nitrite/hr.). Approximately 120 ml/hr. of AA-$H_2O$ was collected overhead. After 5.5 hours of distillation, only a few isolated specks of polymer were observed in the distillation head.

### Example 4 - Preparation and Use of Hydroxyethylammonium Nitrite

Silver nitrite (1g, 0.0065 moles) was dissolved in 175 ml $H_2O$. To this solution was added a mixture of ethanolamine hydrochloride (1.06g of 60 wt. % hydrochloride in $H_2O$, 0.0065 moles) in 25g $H_2O$ dropwise with stirring over a 15 minute period at ambient temperature. A silver chloride precipitate formed immediately. The mixture was stirred for 5 minutes after completion of the addition and then gravity filtered. To 50.6g of the filtrate was added 24.4g of $H_2O$ to produce a 0.24 wt. % aqueous solution of hydroxyethylammonium nitrite. This was used as an inhibitor feed to a batch distillation of AA as described below.

The above prepared solution of 0.24 wt. % hydroxyethylammonium nitrite was fed to the flask at a rate of 10 ml/hr. (0.219 mmole nitrite/hr.). All other conditions of Example 3 were met. A solution of 1.0 wt. % each PTZ and HQ in AA was fed at the top of the distilling head condenser at a rate of 20 ml/hr. (1.04 and 1.35 mmole/hr. of PTZ and HQ, respectively). The flask contents were sparged with 0.05 SCFH of air (13.2 mmole $O_2$/hr.). Approximately 120 ml/hr. of AA-$H_2O$ was collected overhead. After 5.5 hours of distillation, only a few isolated specks of polymer were observed in the distillation head.

### Example 5 - Use of Dicylohexylammonium Nitrite with No Oxygen Sparge

A solution of 0.40 wt. % dicyclohexylammonium nitrite in $H_2O$ was fed to the flask at a rate of 10 ml/hr. (0.187 mmole nitrite/hr.). The conditions were those of Example 1.

A solution of 2.0 wt. % each of PTZ and HQ in AA was fed at the top of the distilling head condenser at a rate of 10 ml/hr. (1.04 and 1.35 mmole/hr. of PTZ and HQ, respectively). The flask contents were sparged with 0.05 SCFH of nitrogen. Approximately 120 ml/hr. of AA-$H_2O$ was collected overhead. No polymer was visible anywhere in the unit after 3.5 hrs. of operation.

### Example 6 - Use of 1-Octyl Nitrite as Vapor Phase Inhibitor

Acrylic acid (600g) is charged to a batch distillation unit consisting of a 5-necked, 1-liter flask surmounted by a 10-plate, 2.54 cm (1 inch) Oldershaw column and distillation head. The acid is distilled at 250 mm system pressure, producing flask and head temperatures of 110 and 84°C, repectively. A solution of 0.28 wt. % 1-octyl nitrite in acrylic acid is fed to the flask at a rate of 10 ml/hr. (1.04 mmole/hr. PTZ and 1.35 mmole/hr. HQ). No air sparge was employed.

Within 45 minutes after the start of distillation a small speck of polymer appeared in a dead space midway up the column. This did not grow with an additional 5 hrs. of distillation.

## Control - Repeat of Example 1 But Without Nitrite Inhibitor

Example 1 was repeated 2 times, except that the aqueous ammonium nitrite feed was omitted. Polymer appeared in the column dead spaces in 49 minutes in one experiment and 79 minutes in the other experiment. (No polymer appeared between the trays of the column, indicating adequate liquid phase inhibition by PTZ and HQ).

## Control - Replace Ammonium Nitrites With Prior Art Nitric Oxide

Example 1 was repeated except that the aqueous ammonium nitrite inhibitor stream was replaced by a sparge of 0.5 wt % nitric oxide in nitrogen at a rate equivalent to 0.189 mmole NO/hr. and the air sparge eliminated. The system was entirely polymer-free after 5.0 hrs. of distillation.

## Control - Repeat of Example 2 But Without Substituted Nitrite Inhibitor

Under conditions similar to Example 2 but with no feed of substituted ammonium nitrite, and with a 10 wt % water in AA feed to the pot of 10 ml/hr., heavy polymer specking had occurred in the distillation head and column dead spaces after one hour. After this time chunks of polymer began dropping out of the head into the column. Polymer growth was rapid and extensive enough to force a shutdown after four hours of operation.

## Control - Repeat of Example 6 But Without Nitrite Inhibitor

In a repeat of Example 6 with the 1-octyl nitrite feed omitted, polymer appeared in vapor areas within 12 minutes and grew continuously over a 2 hr. period.

## Claims

1. A process for inhibiting the formation of unwanted polymer in the vapor phase during the distillation of an unsaturated acid, in which 0.001 to 0.1 weight percent, based on the weight of acid, of non-metallic nitrite salt or nitrite ester is added to the distillation unit during the distillation.

2. A process as claimed in Claim 1 wherein the concentration of nitrite in the vapor phase is from 0.0025 to 0.006 weight percent based on the weight of acid.

3. A process as claimed in Claim 1 or Claim 2 wherein the nitrite comprises $C_6$ to $C_{10}$ alkyl nitrite, for example, octyl nitrite.

4. A process as claimed in any preceding claim wherein the non-metallic nitrite comprises ammonium nitrite, or substituted ammonium nitrite having from 1 to 4 substitutents on the nitrogen atom of the ammonium group, the substitutent(s) being selected from ($C_1$ to $C_{10}$) alkyl, ($C_3$ to $C_6$) cycloalkyl and aryl ($C_1$ to $C_6$) alkyl.

5. A process as claimed in any preceding claim wherein the acid is an alpha, beta unsaturated acid, for example, acrylic or methacrylic acid.

6. A process as claimed in any preceding claim which additionally contains one or more free radical inhibitors in the liquid phase.

7. A process as claimed in Claim 6 wherein the free radical inhibitor is selected from phenothiazine, hydroquinone, and aryl hydroquinones.

8. A process as claimed in Claim 6 or Claim 7 wherein the mole ratio of free radical inhibitor to nitrite is at least 1:1.

9. A process as claimed in Claim 8 wherein the mole ratio of free radical inhibitor to nitrite is 4:1.

10. The use of a non-metallic nitrite ester or nitrite salt for inhibiting the vapor phase polymerization of unsaturated acid.

| Category | Citation of document with indication, where appropriate, of relevant passages | Relevant to claim | CLASSIFICATION OF THE APPLICATION (Int. Cl.4) |
|---|---|---|---|
| D,A | US - A - 2 874 099 (J.W.MECORNEY) <br> * Claims * <br><br> -- | 1,4 | C 07 C 57/075 <br> C 07 C 51/50 |
| D,A | US - A - 3 987 090 (W.G. RUBER et al.) <br> * Examples 5-10 * <br><br> ---- | 1,3,6-8 | |

**DOCUMENTS CONSIDERED TO BE RELEVANT**

EP 88307119.3

**TECHNICAL FIELDS SEARCHED (Int. Cl.4)**

C 07 C 51/00

C 07 C 57/00

C 07 C 67/00

C 07 C 69/00

The present search report has been drawn up for all claims

| Place of search | Date of completion of the search | Examiner |
|---|---|---|
| VIENNA | 10-11-1988 | HOFBAUER |

EPO Form 1503 03 82